# EUROPEAN PATENT SPECIFICATION

(11) **EP 2 467 053 B1**
(45) Date of publication and mention of the grant of the patent: **03.04.2013**
(21) Application number: 10745452.2
(22) Date of filing: 19.08.2010
(51) Int. Cl.: A61B 3/08, A61B 3/107, A61B 3/113

(54) **APPARATUS AND METHOD FOR AUTOMATICALLY DETERMINING A STRABISMUS ANGLE**
VORRICHTUNG UND VERFAHREN ZUR AUTOMATISCHEN DEFINITION EINES SCHIELWINKELS
APPAREIL ET PROCÉDÉ PERMETTANT DE DÉTERMINER AUTOMATIQUEMENT UN ANGLE DE STRABISME

(30) Priority: 20.08.2009 NL 2003372
(43) Date of publication of application: 27.06.2012
(73) Proprietor: Technische Universiteit Delft, 2628 CN Delft (NL)
(72) Inventor: SCHUTTE, Sander, NL-2628 ED Delft (NL); GEUKERS, Elisabeth, Bouwina, Margaretha, NL-2611 AB Delft (NL); SIMONSZ, Huibert, Jan, NL-3034 PE Rotterdam (NL); VAN DER HELM, Franciscus, Cornelis, Theodorus, NL-2286 MN Rijswijk (NL)
(74) Representative: Van Breda, Jacobus
(86) International application number: PCT/NL2010/050521
(87) International publication number: WO 2011/021936

(56) References cited:
- WO-A1-87/02565
- DE-A1- 19 649 490
- SHIH SHENG-WEN ET AL: "A novel approach to 3-D gaze tracking using stereo cameras." IEEE TRANSACTIONS ON SYSTEMS, MAN, AND CYBERNETICS. PART B, CYBERNETICS : A PUBLICATION OF THE IEEE SYSTEMS, MAN, AND CYBERNETICS SOCIETY FEB 2004, vol. 34, no. 1, February 2004 (2004-02), pages 234-245, XP002574761 ISSN: 1083-4419 cited in the application

## Description

The invention relates to an apparatus and a method for determining a strabismus angle of the eyes of an individual by performing a reflection test on said eyes.

Such an apparatus and method are known from US-A-5,502,520 and from DE-A-196 49 490.

Strabismus is a frequent disorder among children, affecting approximately 3-4% of the population. Strabismus is a misalignment of the eyes in which the visual axes are not directed to the same fixation point. Except from the cosmetic effects, strabismus may cause amblyopia (a 'lazy eye') in children and diplopia (double vision) in adults. Strabismus is usually corrected by muscle surgery, which aims to correct the alignment of the eyes, by relocating the eye muscles with shortening or lengthening one or two eye muscle attachments to the eye, during eye muscle surgery.

The reoperation rate for strabismus surgery is between 20% and 50% in children operated under the age of four. This puts a burden on costs of healthcare and the quality of life of patients. Strabismus operations in the Netherlands are performed roughly 150 times a week. More than half of the strabismus reoperations can be attributed to three sources of variance: (1) inaccurate preoperative measurements of the strabismus angle (20% of the reoperations), (2) variability in surgical strategy among ophthalmologists and orthoptists (15%) and (3) inaccurate surgery (20%). The remaining variance is attributable to individual differences in recovery, anatomy and physiology of the patient's eye muscles or orbit and differences in degree of binocular vision.

In the apparatus and method of DE-A-196 49 490 a strabismus angle of the eyes of an individual is determined by performing a reflection test on said eyes, comprising the steps of:
- directing at least a first beam of light onto said eyes of the individual;
- having the individual focus its eyes;
- using an imaging device for performing a reflex test on both eyes to estimate the strabismus angle,
   wherein:
- the reflex test is carried out by applying at least two light sources at known differing positions and measuring the corneal reflections of said light sources in both eyes by monitoring said corneal reflections by an imaging device or devices;
- the said corneal reflections are used to derive corneal center coordinates for both eyes individually;
- for both eyes individually pupil center coordinates are estimated; and
- the corneal center coordinates and the pupil center coordinates of both eyes are used to estimate the strabismus angle.

In the apparatus and method known from US-A-5,502,520 the following steps are applied:
- directing at least a first beam of light onto the eyes of the individual;
- having the individual focus its eyes on the first beam of light;
- using at least one imaging device for measuring the light beam reflected from the eyes for determining a Brückner reflex test and performing a Hirschberg reflex test. The reflected light beam is for this purpose recorded with a video apparatus, and the recorded images are later forwarded to an eye specialist for a diagnosis of eye disorders indicated by the said Brückner and Hirschberg reflex tests. Although this known apparatus and method obviate the need of the presence of the eye specialist, and allow that any medical practitioner can take the test, it is still disadvantageous in that it needs the efforts of the eye specialist to analyze in a conventional manner the presence of eye disorders, such as in particular strabismus.

The problems that attach to the conventional manner of determining and treating strabismus and which cause the high rate of reoperations as discussed above, therefore still remain.

Another drawback of the prior art relates to the conventional use of the Hirschberg-test. This test is based on determining the position of the corneal light reflection (first Purkinje image) relative to the pupil center when a light is shone onto the eyes. If the eyes are aligned properly, the reflection will appear on the same (mirrored) location on each eye. If the patient is strabismic, the reflections will be asymmetrical. The angle of strabismus is evaluated from the misalignment of the reflection in the strabismic eye. Because of its dependency on a patient's anatomy such as its corneal curvature, which varies significantly and is not individually estimated, the Hirschberg-test is unreliable with a reported standard deviation of 3.9°, see Hasebe, S. et al, The reliability of a video-enhanced Hirschberg-test under clinical conditions. Invest Ophtalmol Vis Sci, 1995. 36(13), pages 2678-85.

Latent strabismus angles are currently measured with a standard deviation of 1.7° and manifest strabismus with a standard deviation between 3.9 and 5.4°. The average strabismus assessment lasts a quarter of an hour if the angle of strabismus is measured in all gaze directions, and requires cooperation of a patient. This presents major problems in patients below four years old, which concerns the majority of the patients. Children, more than adults, require a fast, objective and entertaining measurement approach to measure strabismus angles. A child's concentration span is limited (in the order of seconds) and generally decreases during a task. Due to the limitations of the current measurement methods there is a need for a new method and apparatus to measure strabismus angles (particularly with children) in a quick and accurate way.

In summary the main drawbacks of the current state of the art to measure strabismus angles are its limited accuracy, the time required to obtain a measurement and its dependency on patient cooperation.

It is therefore one of the objectives of the invention to propose a method and apparatus with which it is possible to assess the angle of strabismus in a quick (within 30 seconds) and accurate (< 0.5° SD) way, by using a technique that minimally relies on the cooperation of the (young) patient. Further it is an objective of the invention to be able to measure from a distance of at least one meter, and in a manner that free head movement is allowed. By using an objective and accurate measurement method, the inter- and intra-observer variations in the preoperative measurement can be reduced and young uncooperative patients can be measured more easily. Moreover, an easier and faster measurement method offers the possibility to assess a patient's strabismus angle more frequently. This way, the intra-patient variability can be taken into account in the decisions for surgery.

In order to meet one or more of the above objectives, the method and apparatus of the invention are characterized by the independent claims.

In a first aspect of the invention, a method for automatically determining a strabismus angle of the eyes of an individual is proposed wherein:
- first the light beam reflected from the eyes is measured in a first test for establishing which eye of both eyes is fixating, and that thereafter:
- for both the fixating eye and the non-fixating eye individually the estimated corneal center coordinates and the estimated pupil center coordinates are used to establish each eye's optical axis through the corneal center and the pupil center of the concerning eye; and that
- for the eye that is fixating the angle kappa is established between the fixating eye's optical axis and a first visual axis through that fixating eye's corneal center and its fovea;
- for the non-fixating eye a second visual axis is derived from that non-fixating eye's optical axis and the angle kappa that is established for the fixating eye;
- and that the strabismus angle is derived from the so derived first and second visual axes of both eyes.

In this manner it is possible to automate the determination of the strabismus angle and exclude the necessity from the prior art to include a human determination by an eye specialist of the concerning eye disorder. Accordingly it is possible to improve the speed, reliability and accuracy of the determination of the strabismus angle. The method further provides the advantageous feature that the strabismus angle can be measured in all directions of gaze, and not merely in a 2D-plane. A further advantage of the invention is that the strabismus-angle can be measured as a function of the position of the individual's head.

The method of the invention can effectively be carried out in an apparatus having the features that it comprises at least two light sources at different positions, that its imaging device is arranged to measure the corneal reflections of said light sources in both eyes, and that it comprises calculating means connected to the imaging device that is arranged to effect that
- the said corneal reflections are used to derive corneal center coordinates for both eyes individually;
- for both eyes individually pupil center coordinates are estimated; and that
- the corneal center coordinates and the pupil center coordinates of both eyes are used to estimate the strabismus angle.

According to the invention this apparatus has the following features, notably an imaging device arranged to measure a light beam reflected from the eyes for establishing which eye of both eyes is fixating, and calculating means arranged to determine:
- for both the fixating eye and the non-fixating eye individually the estimated corneal center coordinates and the estimated pupil center coordinates, and to establish for both the fixating eye and the non-fixating eye the optical axis through the corneal center and the pupil center of the concerning eye;
- for the eye that is fixating the angle kappa between the fixating eye's optical axis and a first visual axis through that fixating eye's corneal center and its fovea;
- for the non-fixating eye a second visual axis depending on that non-fixating eye's optical axis and the angle kappa that is established for the fixating eye; and
- the strabismus angle from the so derived first and second visual axes of both eyes.

In order to promote the operational speed of the apparatus and the method applied thereby, it is advantageous that the imaging device is arranged for stereo imaging. This can effectively be implemented by arranging that the imaging device comprises two cameras at different locations.

The invention will hereinafter be further elucidated with reference to the following discussion of a method in accordance with the invention, and a provisional set up of an apparatus in accordance with the invention.

In the following discussion reference will further be made to the drawing, in which:
- figure 1 shows schematically two eyes and the relevant optical and visual axes;
- figure 2 shows schematically the calculation scheme for the strabismus angle in accordance with the invention;
- figure 3 shows a photograph demonstrating asymmetry in the reflexes of the eyes attributable to strabismus;
- figure 4 shows schematically a first test as applied in accordance with the invention;
- figure 5 shows a stereo camera setup for application in the method of the invention;
- figure 6 provides an exemplary image of the eyes of an individual showing three corneal reflections;
- figure 7 shows schematically the measurement protocol of the automated strabismus measurement in accordance with the invention;
- figure 8 shows schematically a ray diagram of one light source reflected in a convex mirror and observed by two cameras;
- figure 9 shows schematically a ray diagram with three light sources reflected in a convex mirror and observed by two cameras;
- figure 10 shows the virtual image of the pupil, as observed by two cameras;
- figure 11 shows a schematic top view of an eye with its concerning optical and visual axes; and
- figure 12 shows the experimental setup of the apparatus in accordance with the invention.

The measurement method of the invention will be discussed with reference to the following definitions which generally apply in the field.

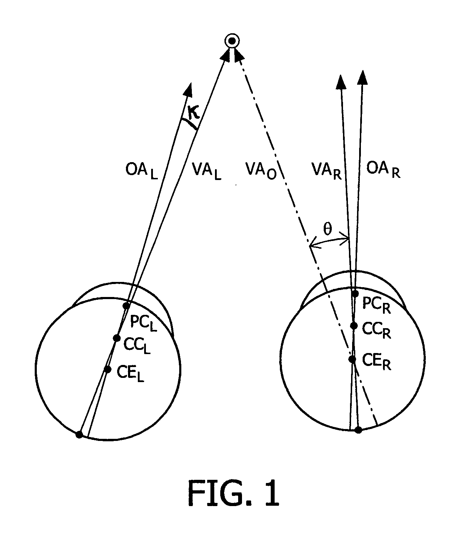

In orthotropic subjects, the visual axes (VA) of both eyes are directed at the fixation target (see Fig.1). A strabismus patient fixates with only one eye at a time.

In figure 1 the following legend applies:
OA = optical axis fixation target
VA= visual axis
VAO = visual axis in orthotropic
PC = pupil center
CC = corneal sphere center
CE = eyeball center

Fig.1 shows the strabismus angle theta defined as the angle between the visual axis of the strabismic eye and the axis where the eye is supposed to point at in the situation of binocular fixation. The optical axis (OA) and visual axis (VA) of both eyes are represented. CC is the corneal center. CE represents the (rotation) center of the eye. In figure 1, the left eye fixates on the fixation target and the right eye is deviated outward (exodeviation).

The visual axis (VA) of the human eye originates as figure 1 shows from the fovea, crosses the corneal sphere center (CC) and is directed towards a fixation object [23]. The visual axis does generally not coincide with the optical axis of the human eye. The fovea, where the visual axis originates does not have a central position on the retina, but is located 3.9 ± 2.7° to the temporal side. The optical axis (OA) is considered the symmetry axis of the eye and is assumed to cross three principal points of the eye: the pupil center PC, the corneal center CC and the eye center CE.

As mentioned earlier the angle kappa is defined as the angle between the visual axis and the optical axis. Kappa is highly correlated in both eyes (r=0.8996 [24]), although sometimes asymmetries exist. Since its variability is high, a calibration is needed before an accurate measurement can be obtained.

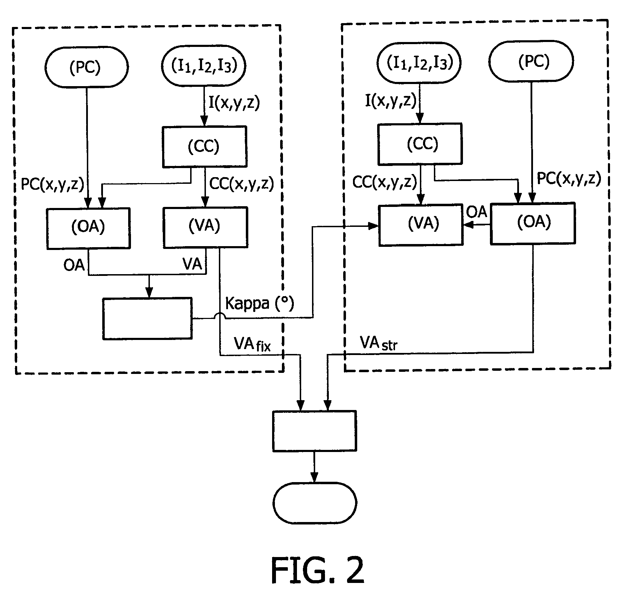

The angle kappa can be estimated in the patient's fixating eye with a calibration, when the subject instantaneously fixates on the coaxial light source. Kappa is assumed to be equal in both eyes. To compute the strabismus angle the visual axes (VA) in both eyes have to be determined. In the fixating eye, the visual axis is assumed to point at the fixation target. In the strabismic eye, the visual axis can not be found directly, but is projected on the found optical axis, by angle kappa (see also the schematic calculation scheme of Fig.2).

The measurement protocol in accordance with the invention to determine a patient's strabismus angle is discussed in the following with reference to figure 2. In figure 2 square blocks represent calculation processes, rounded blocks represent input and output data.

In the calculation scheme for the strabismus angle as shown in figure 2, first the angle kappa is calibrated in the dominant (fixating) eye. The corneal reflection centers 11, 12, 13 and, pupil center are determined in 3D coordinates. Then the center of corneal curvature CC is calculated, and subsequently the optical axis (OA) and visual axis (VA) in the fixating eye are determined. In the strabismic eye, only the optical axis (OA) can be measured.

As discussed above the angle kappa is the angle between the optical and visual axes and is assumed to be equal in both eyes. Consistent therewith for the strabismic eye the visual axis is projected on the OA by angle kappa. The strabismus angle is then calculated with the VA in the fixating eye and VA in the strabismic eye.

Hereinafter a detailed disclosure of each method step in accordance with the invention will be provided.

### Step 1: Detection of the fixating eye

The first step in assessing the strabismus angle is the determination of the fixating eye. The question whether a patient fixates with its right or left eye can for instance be solved with the Brückner test.

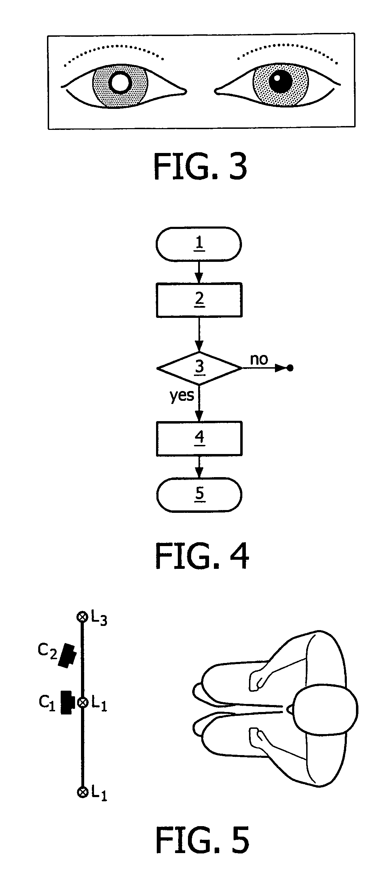

Brückner (1962)[25] introduced the test, in which a bright coaxial light source is used to illuminate both eyes of the subject. Two red fundus reflexes appear and the brightness of these reflexes is compared. When the light shines exactly in the fovea a darker response is seen than when the light is reflected by the periphery of the fovea. Strabismus or anisometropia can be diagnosed from the asymmetry of these reflexes without touching the child (see Fig.3).

Fig.3 shows the asymmetry of the red reflexes attributable to (micro)strabismus. The pupil of the patient's fixating eye (right in the image) appears darker than the strabismic eye. Figure 3 is adapted from [27].

By using a light source that is exactly coaxial with a camera, an automated test can be performed. The Brückner test is sensitive to anisometropia (the condition in which the two eyes have unequal refractive power) in the assessment of pupil brightness [13]. This causes false positives for strabismus and should be taken into account, for instance by assessing the refractive errors should be assessed prior to performing the test.

In the method and apparatus of the invention an automated version of the test is implemented as schematically shown in Fig.4.

As an input to the test as represented by block 1 first the segmented pupils are acquired from the obtained images. Block 2 then calculates and compares the average pupil brightness. Block 3 redoes the test if the pupil brightness of both pupils is larger than a fixation threshold of 1° to 2°, else it continues to block 4. In block 4 the dominant (fixating) eye is determined by the lowest pupil brightness. Finally in block 5 it provides the output: the patient fixates and its fixating eye is L/R.

When fixation is assured and the fixating eye is determined, the visual axis (VA) in this eye can be derived.

### Step 2: Individual parameter estimation through a one-point calibration

To determine the rotation in both eyes, in the method of the invention an automated technique is applied.

A person's eye rotation can be derived from the pupil centers and corneal reflections (hereinafter referred to as PCCR).

The PCCR technique is related to the Hirschberg test according to the prior art to determine strabismus angles. The human cornea acts like a convex mirror: when a light is shone onto the eye, a reflection (first Purkinje image) appears on the cornea. The horizontal or vertical distance between the center of this reflection and the center of a person's pupil can be used as a measure for the eye rotation.

The PCCR technique can be applied in non-cooperative patients and requires at least a setup with one camera and one light source. The main disadvantage in this configuration, however, is its sensitivity to human biometric parameters. A person's radius of corneal curvature (Rc) and the distance between the light source, camera and the subject strongly affect the position where the corneal reflection appears. The position of the observed pupil center is affected by refraction of the cornea and the anterior chamber depth (ACD); the position of the real pupil. A patient's Rc and ACD have an influence on the measurement accuracy of PCCR and vary significantly [6, 24].

A fourth parameter that influences the accuracy of the PCCR is a patient's angle kappa, the angle between the visual axis (VA) and the optical axis (OA). Before an accurate measurement can be performed, an individual calibration of these parameters should be done. In the PCCR with one camera and one light source, the subject is therefore generally asked to fixate on a number of calibration points to estimate the parameters Rc, ACD and the angle kappa.

In order to circumvent the Rc and ACD need to apply more than one calibration point, it is in accordance with the invention to apply an arrangement as shown in figure 5 with a stereo camera setup with two cameras (C1, C2) and at least two, and preferably at least three infrared light sources (L1, L2, L3). In this setup it is possible to apply a method avoiding calibrating a person's corneal radius and anterior chamber depth.

With the stereo camera setup as shown in figure 5, the corneal reflections and pupil centers can be calculated in 3D global coordinates. With multiple light sources it is possible to calculate the center of the cornea CC, assuming that the cornea -as mentioned above- behaves as a convex mirror. This way, the optical axis (OA) and visual axis (VA) (as discussed with reference to Fig.1) of both eyes of a patient can be determined in 3D, which will be explained in detail hereinafter. The angle kappa can thus be calibrated with one-point fixation on a coaxial light source, which can also be used to perform the Brückner test.

This one-point calibration requires only little time and cooperation from the individual subjected to the method, which is particularly advantageous with a young child. In the designed setup as shown in figure 5, a stereo camera setup is used, in which the two cameras are considered to act as pinhole cameras. The light sources are considered point sources.

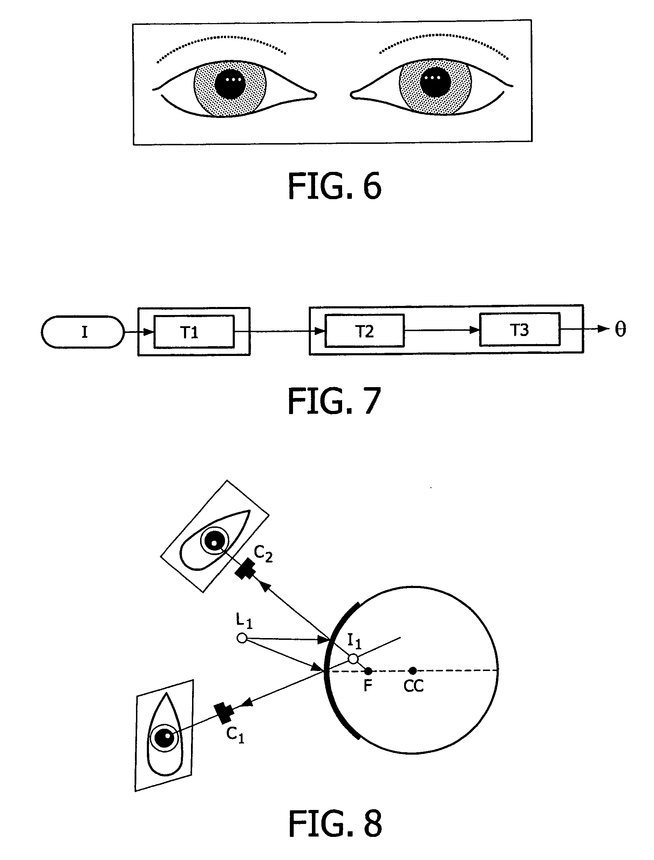

Fig.6 provides an example image of an exotropic subject observed by camera 2 (Fig.5). The three corneal reflections (first Purkinje images) of the three light sources are visible on the subject's pupils.

### Measurement calculations

To calculate the strabismus angle in a patient, the optical axes (OA) and visual axes (VA) in both eyes are estimated from the pupil centers and corneal reflections. The center of corneal curvature CC (Fig.1), which is part of both OA and VA, is calculated for both eyes, using the corneal reflections (Fig.6), which are virtual images behind the corneal surface.

### Pupil Center PC

The optical axis (OA) of the eye can be determined with the information of nodal point CC and the location of the pupil center PC. When an eye is observed by a camera, the image displays the virtual image of the pupil, instead of the physical pupil. The virtual image of the pupil center (PC) differs from the physical pupil center (PP), due to the refraction of the cornea, and will be seen magnified and displaced anteriorly.

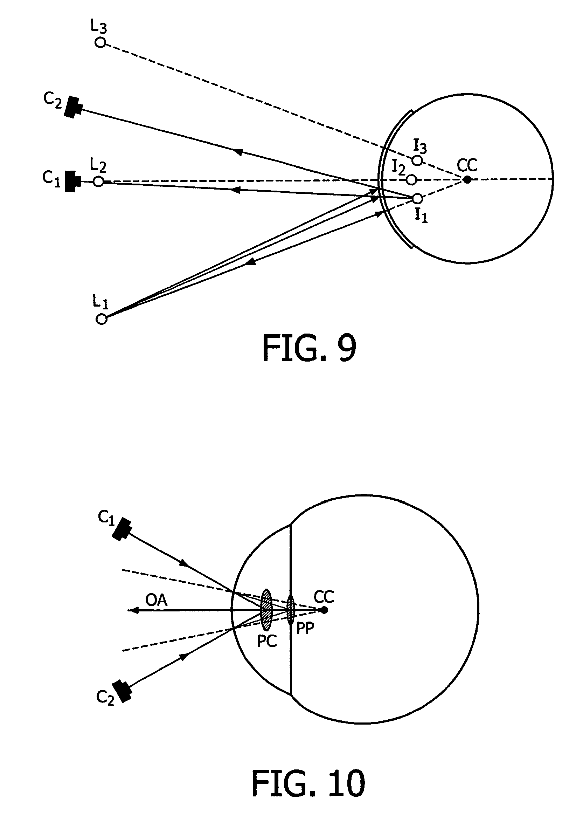

The virtual image of the pupil is independent of the camera position and can be assumed to lie on the same optical axis as the physical pupil center PP. When observed by a stereo camera setup, the 3D location of the virtual image of the pupil can be determined by a 3D triangulation, as can be seen in Fig.10.

Fig.10 shows a virtual image of the pupil, observed by two cameras. CC represents the corneal center of curvature, PP the physical pupil center and PC the used virtual pupil center.

### Optical and visual axes in the fixating eye

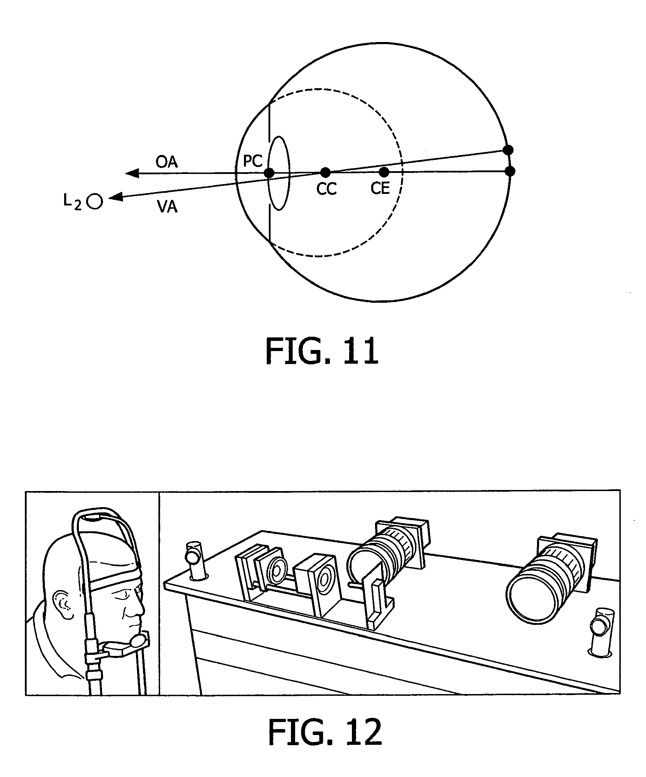

By knowing the 3D position of the fixation target (e.g. the coaxial light source L2) and the corneal center CC, the direction of the visual axis VA of the fixating eye (Fig.11) can be calculated. The optical and visual axes in the fixating eye are determined and the angle kappa is calculated as the angle between these two axes in horizontal and vertical directions (in the reference frame of the head).

Fig.11 shows schematically a top view of the right eye of the human eye with the visual axis VA, originating from the fovea, crossing CC and directed to the fixation target. The optical axis OA crosses CC and pupil center PC. Angle kappa is the angle between OA and VA. CC is the center of corneal curvature and CE the eye ball center [32].

### Optical and visual axes in the strabismic eye

The optical axis of the strabismic (non-fixating) eye (OAstr) can be determined also by deriving the pupil center PC and corneal center CC. With the assumption that angle kappa is equal in both eyes, the visual axis of the strabismic eye (VAstr) is revealed by a vector projection on its optical axis.

When the visual axis in the strabismic eye is calculated, the horizontal strabismus angle is determined as the angle in horizontal direction between the strabismic visual axis and the reference axis where the eye was supposed to point at (Fig. 1) If desired the vertical angle of strabismus can be computed as well.

### Corneal Center CC

When a ray of light is reflected on a convex surface, such as the cornea, the reflection (or first Purkinje image) is formed as a virtual image behind the surface (see Fig.8). The corneal surface is assumed to be spherical. The reflection law of convex mirrors establishes that the virtual light position is only determined by the actual position of the light source and by the position of the mirror, independent of the camera position [32]. If two cameras of a stereo camera setup are placed at different locations, each camera will capture the same virtual image of the light source in space.

With the use of a stereo camera setup the 3D location of the virtual image of the corneal reflection can be calculated according to the theory of convex mirrors.

According to the properties of a convex mirror an incident ray that is directed towards the center of curvature of the mirror, is reflected back along its own path. The virtual image of the reflection of a light source will lie in this path. The light source, the virtual image of its corneal reflection, and the center of curvature of the cornea will be colinear [32]. If at least two light sources with known positions are used, this colinearity can be used to calculate the corneal center of curvature CC (see also Fig.9) by determining the points of intersection of the obtained lines. The accuracy of this determination can be improved by increasing the number of light sources.

Fig.9 shows a setup that can be used for the one-point calibration with two cameras (C1 and C2) and three light sources (L1, L2, L3), generating 3 virtual images (I1, I2, I3) of corneal reflections. CC represents the corneal center. L, I and CC are colinear.

### Step 3: Strabismus angle measurement

Once the visual axes (VA) have been determined in the fixating and strabismic eye in the manner described above, it is possible to derive both in vertical and horizontal direction the quantitative strabismus angle of a patient.

The total scheme to determine a patient's fixating eye, to estimate a patient's angle and to calculate the strabismus angle, is represented in Fig.7.

Fig.7 shows the scheme of the measurement protocol of an automated strabismus measurement in accordance with the invention. A first test T1 is used to determine from acquired images I the patient's fixating (dominant) eye (step 1). The angle kappa is calibrated with a one-point calibration T2 on a fixation target (step 2). Then in step 3 represented by block T3 the strabismus angle can be straightforwardly computed.

The calculations done to perform the quantitative measurement of the strabismus angle are described in detail in the previous section. A detailed calculation process scheme is represented in Fig.2.

### Experimental setup of an apparatus in accordance with the invention

A schematical view of the experimental setup that is used to demonstrate the invention is shown in Fig. 12. Two synchronized ethernet Prosilica cameras (GC2450) with a resolution of 2450x2050 pixels have been mounted on a tripod. Two lenses with 75 mm focal length have been mounted, aimed to measure patients at 1.1 m distance from the camera with a field of view of 120mm. The cameras have been calibrated with a calibration toolbox in Matlab [33].

Fig.12 shows on the left: A picture of an individual with his head placed in a chin rest. On the right figure 12 shows the used experimental configuration with the stereo camera setup and three (IR) light sources of which one was coaxial with the left camera.

To invoke the corneal reflections three infrared light sources (Epigap) with a wavelength of 870 nm and a radiant power of 240 mW have been applied in an array. Infrared light is used because visible light reflections may be confused with ambient disturbing light reflections. For the fixation target, light in the visible spectrum was used.

On the camera lenses, two infrared transmitting filters were mounted. The infrared-transmitting filters absorb most of the visible light region and transmit the infrared region (> 700 nm). The light sources are completely safe for the human eye. Two light sources (L1 and L3) are pointed directly to the subject, whereas the central light source (L2) is focused by a lens on a small aluminium coated mirror under 45° in front of one camera to invoke the Bruckner reflex and to serve as a fixation target for the one-point calibration.

For the image processing and the extraction of the centers of the corneal reflections and the pupil centers, a Starburst algorithm [34] was implemented. Starburst is an open-source eye-tracking algorithm in Matlab, originally developed for head-mounted eye-tracking systems [34]. The algorithm begins by locating and removing the corneal reflections from the image. The center of the corneal reflection is computed as the gravity center of the bright reflection. The pupil edge points are located using an iterative feature-based technique. An ellipse is fitted to the detected edge points [35]. Each time the algorithm is performed, the result is a set of three corneal reflection centers and 1 pupil center in 2D picture coordinates (see Fig.13).

When the pupil centers and corneal reflections of both eyes are extracted from the images of the two cameras, a stereo triangulation is performed to calculate the 3D coordinates of the respective points in space.

### Bibliography

1. Greenberg, A.E., B.G. Mohney, N.N. Diehl, et al., Incidence and types of childhood esotropia: a population-based study. Ophthalmology, 2007. 114(1): p. 170-4.
2. Govindan, M., B.G. Mohney, N.N. Diehl, et al., Incidence and types of childhood exotropia: a population-based study. Ophthalmology, 2005. 112(1): p. 104-8.
3. Kvarnstrom, G., P. Jakobsson, and G. Lennerstrand, Visual screening of Swedish children: an ophthalmological evaluation. Acta Ophthalmol Scand, 2001. 79(3): p. 240-4.
4. Simonsz, H.J., G.H. Kolling, and K. Unnebrink, Final report of the early vs. late infantile strabismus surgery study (ELISSS), a controlled, prospective, multicenter study. Strabismus, 2005. 13(4): p. 169-99.
5. Schutte, S., J.R. Polling, F.v.d. Helm, et al., Human Error in Strabismus Surgery: Quantification with a Sensitivity Analysis. 2008.
6. Hasebe, S., H. Ohtsuki, R. Kono, et al., Biometric confirmation of the Hirschberg ratio in strabismic children. Invest Ophthalmol Vis Sci, 1998. 39(13): p. 2782-5.
7. Hasebe, S., H. Ohtsuki, Y. Tadokoro, et al., The reliability of a video-enhanced Hirschberg test under clinical conditions. Invest Ophthalmol Vis Sci, 1995. 36(13): p. 2678-85.
8. Ruff, H.A.L., Katharine R., Development of sustained, focused attention in young children during free play. Developmental Psychology, 1990. 26(1): p. 85-93.
9. Barry, J.C., R. Effert, A. Kaupp, et al., Measurement of ocular alignment with photographic Purkinje I and IV reflection pattern evaluation. Invest Ophthalmol Vis Sci, 1994. 35(13): p. 4219-35.
10. Barry, J.C., R. Effert, M. Reim, et al., Computational principles in Purkinje I and IV reflection pattern evaluation for the assessment of ocular alignment. Invest Ophthalmol Vis Sci, 1994. 35(13): p. 4205-18.
11. Barry, J.C., A. Backes, U.M. Pongs, et al., Improved computing scheme for measuring eye alignment with Purkinje images I and IV. Ophthalmic Physiol Opt, 1997. 17(5): p. 433-40.
12. Barry, J.C., R. Effert, and A. Kaupp, Objective measurement of small angles of strabismus in infants and children with photographic reflection pattern evaluation. Ophthalmology, 1992. 99(3): p. 320-8.
13. Cibis, G.W., Video vision development assessment (VVDA): combining the Bruckner test with eccentric photorefraction for dynamic identification of amblyogenic factors in infants and children. Trans Am Ophthalmol Soc, 1994. 92: p. 643-85.
14. Hunter, D.G., D.S. Nassif, N.V. Piskun, et al., Pediatric Vision Screener 1: instrument design and operation. J Biomed Opt, 2004. 9(6): p. 1363-8.
15. Hunter, D.G., S.N. Patel, and D.L. Guyton, Automated detection of foveal fixation by use of retinal birefringence scanning. Applied Optics, 1999. 38(7): p. 1273-1279.
16. Nassif, D.S., N.V. Piskun, B.I. Gramatikov, et al., Pediatric Vision Screener 2: pilot study in adults. J Biomed Opt, 2004. 9(6): p. 1369-74.
17. Nassif, D.S., N.V. Piskun, and D.G. Hunter, The Pediatric Vision Screener III: detection of strabismus in children. Arch Ophthalmol, 2006. 124(4): p. 509-13.
18. Ohno, T. and N. Mukawa, A Free-head, Simple Calibration, Gaze Tracking System That Enables Gaze-Based Interaction. ACM, 2004: p. 115-122.
19. Shih, S.W. and J. Liu, A novel approach to 3-D gaze tracking using stereo cameras. IEEE Trans Syst Man Cybern B Cybern, 2004. 34 (1) : p. 234-45.
20. Young, L.R. and D. Sheena, Eye-movement measurement techniques. Am Psychol, 1975. 30(3): p. 315-30.
21. Duchowski, A., Eye Tracking Methodology; Theory and practice. 2nd ed. 2007: Springer.319.
22. Glenstrup, A.J. and T. Engell-Nielsen, Eye Controlled Media: Present and Future State. 1995, DIKU, University of Copenhagen: Copenhagen. p. 1-82.
23. Villanueva, A. and R. Cabeza, Models for Gaze Tracking Systems. EURASIP Journal on Image and Video Processing, 2007.
24. Schaeffel, F., Kappa and Hirschberg ratio measured with an automated video gaze tracker. Optom Vis Sci, 2002. 79(5): p. 329-34.
25. Brückner, R., Exacte Strabismusdiagnostik bei 1/2-3 jahrigen Kindern mit einem einfachen Verfahren, dem 'Durchleuchtungstest'. Ophthalmologica, 1962(144): p. 184198.
26. Carrera, A., M.A. Soarnil, and M.I. Zamora, Detecting ablyogenic diseases with the photographic Bruckner test. Strabismus, 1993. 1(1): p. 3-9.
27. Paysse, E.A., G.C. Williams, D.K. Coats, et al., Detection of red reflex asymmetry by pediatric residents using the Bruckner reflex versus the MTI photoscreener. Pediatrics, 2001. 108(4): p. E74.
28. Kaakinen, K., A simple method for screening of children with strabismus, anisometropia or ametropia by simultaneous photography of the corneal and the fundus reflexes. Acta Ophthalmol (Copenh), 1979. 57(2): p. 161-71.
29. Miller, J.M., H.L. Hall, J.E. Greivenkamp, et al., Quantification of the Bruckner test for strabismus. Invest Ophthalmol Vis Sci, 1995. 36(5): p. 897-905.
30. Guestrin, E.D. and M. Eizenman, General theory of remote gaze estimation using the pupil center and corneal reflections. IEEE Trans Biomed Eng, 2006. 53(6): p. 1124-33.
31. Guestrin, E.D. and M. Eizenman, Remote point-of-gaze estimation with free head movements requiring a single-point calibration. Conf Proc IEEE Eng Med Biol Soc, 2007. 2007: p. 4556-60.
32. Zhu, Z. and Q. Ji, Novel eye gaze tracking techniques under natural head movement. IEEE Trans Biomed Eng, 2007. 54(12): p. 2246-60.
33. Bouguet, J.-Y. Camera Calibration Toolbox for Matlab. 2008 [cited; Available from:
   http://www.vision.caltech.edu/bouguetj/calib_doc/.
34. Li, D. and D.J. Parkhurst, Starburst: A robust algorithm for video-based eye tracking. Elsevier Science, 2005. (Preprint).
35. Fischler, M.A. and R.C. Bolles, Random Sample Consensus: A paradigm for Model Fitting with Applications to Image Analysis and Automated Cartography. ACM, 1981. 24(6): p. 381-395.

## Claims

1. Method for automatically determining a strabismus angle of the eyes of an individual by performing a reflection test on said eyes, comprising the steps of:
- directing at least a first beam of light onto said eyes of the individual;
- having the individual focus its eyes;
- using an imaging device for performing a reflex test on both eyes to estimate the strabismus angle,
wherein:
- the reflex test is carried out by applying at least two light sources at known differing positions and measuring the corneal reflections of said light sources in both eyes by monitoring said corneal reflections by an imaging device or devices;
- the said corneal reflections are used to derive corneal center coordinates (GG₂, GG_{R}) for both eyes individually;
- for both eyes individually pupil center coordinates (PG_{L}, PG_{R}) are estimated; and
- the corneal center coordinates and the pupil center coordinates of both eyes are used to estimate the strabismus angle,
- first the light beam reflected from the eyes is measured in a first test for establishing which eye of both eyes is fixating, and thereafter:
- for both the fixating eye and the non-fixating eye individually the estimated corneal center coordinates and the estimated pupil center coordinates are used to establish each eye's optical axis (OA_{L,} OA_{R}) through the corneal center and the pupil center of the concerning eye;
- for the eye that is fixating the angle kappa is established between the fixating eye's optical axis and a first visual axis (VA_{L}) through that fixating eye's corneal center and its fovea;
- for the non-fixating eye a second visual axis (VA_{R}) is derived from that non-fixating eye's optical axis and the angle kappa that is established for the fixating eye;
- the strabismus angle is derived from the so derived first and second visual axes of both eyes.

2. Method according to claim 1, wherein monitoring of the corneal reflections by the imaging device or devices is performed by using at least two cameras at different locations.

3. Method according to claim 1 or 2, wherein the position of the individual's head is measured, and the strabismus-angle is related to said position.

4. Apparatus for automatically determining a strabismus angle of the eyes of an individual, comprising:
- at least a first light source (L₁) for directing light onto the eyes of the individual,
- an imaging device (G₁, G₂) for performing a reflex test on both eyes to estimate the strabismus angle, and further comprising:
- two further light sources (L₂, L₃) at different positions, wherein the imaging device is arranged to measure the corneal reflections of said light sources in both eyes,
- calculating means connected to the imaging device so as to effect that:
- the said corneal reflections are used to derive corneal center coordinates (GG_{L}, GG_{R}) for both eyes individually;
- for both eyes individually pupil center coordinates (PG_{L}, PG_{R}) are estimated;
- the corneal center coordinates and the pupil center coordinates of both eyes are used to estimate the strabismus angle,
wherein
- the imaging device is arranged to measure the light beam reflected from the eyes for establishing which eye of both eyes is fixating, and
- the calculating means is arranged to determine:
.for both the fixating eye and the non-fixating eye individually the estimated corneal center coordinates and the estimated pupil center coordinates, and to establish for both the fixating eye and the non-fixating eye the optical axis (OA_{L}, OA_{R}) through the corneal center and the pupil center of the concerning eye;
. for the eye that is fixating the angle kappa between the fixating eye's optical axis and a first visual axis (VA_{L}) through that fixating eye's corneal center and its fovea;
.for the non-fixating eye a second visual axis (VA_{R}) depending on that non-fixating eye's optical axis and the angle kappa that is established for the fixating eye;
.the strabismus angle from the so derived first and second visual axes of both eyes.

5. Apparatus according to claim 4, wherein the imaging device is arranged for stereo imaging.

6. Apparatus according to claim 5, wherein the imaging device comprises at least two cameras at different locations.

## Patentansprüche

1. Verfahren zum automatischen Bestimmen eines Strabismuswinkels der Augen einer Person durch Ausführen eines Reflexionstests an den Augen, umfassend folgende Schritte:
- Richten mindestens eines ersten Lichtstrahls auf die Augen der Person;
- Veranlassen, dass die Person ihren Blick einstellt;
- Verwenden einer Bildgebungsvorrichtung zum Ausführen eines Reflextests an beiden Augen, um den Strabismuswinkel zu schätzen,
wobei:
- der Reflextest ausgeführt wird, indem mindestens zwei Lichtquellen an bekannten unterschiedlichen Positionen angelegt werden und die Hornhautreflexionen der Lichtquellen in beiden Augen gemessen werden, indem die Hornhautreflexionen durch eine oder mehrere Bildgebungsvorrichtungen kontrolliert werden;
- die Hornhautreflexionen verwendet werden, um die Koordinaten (CC_{L}, CC_{R}) des Hornhautmittelpunktes für beide Augen einzeln abzuleiten;
- für beide Augen die Koordinaten (PC_{L}, PC_{R}) des Pupillenmittelpunktes einzeln geschätzt werden; und
- die Koordinaten des Hornhautmittelpunktes und die Koordinaten des Pupillenmittelpunktes der beiden Augen verwendet werden, um den Strabismuswinkel zu schätzen,
- erst der Lichtstrahl, der von den Augen reflektiert wird, in einem ersten Test gemessen wird, um festzustellen, welches Auge der beiden Augen fixiert, und anschließend:
- sowohl für das fixierende Auge als auch für das nicht fixierende Auge einzeln die geschätzten Koordinaten des Hornhautmittelpunktes und die geschätzten Koordinaten des Pupillenmittelpunktes verwendet werden, um die optische Achse (OA_{L}, OA_{R}) jedes Auges durch den Hornhautmittelpunkt und den Pupillenmittelpunkt des betreffenden Auges festzustellen;
- für das fixierende Auge der Winkel Kappa zwischen der optischen Achse des fixierenden Auges und einer ersten Sichtachse (VA_{L}) durch den Hornhautmittelpunkt dieses fixierenden Auges und seine Sehgrube festgestellt wird;
- für das nicht fixierende Auge eine zweite Sichtachse (VA_{R}) aus der optischen Achse dieses nicht fixierenden Auges und dem Winkel Kappa, der für das fixierende Auge festgestellt wurde, abgeleitet wird;
- der Strabismuswinkel aus den so abgeleiteten ersten und zweiten Sichtachsen der beiden Augen abgeleitet wird.

2. Verfahren nach Anspruch 1, wobei die Kontrolle der Hornhautreflexionen durch die Bildgebungsvorrichtung(en) durchgeführt wird, indem mindestens zwei Kameras an verschiedenen Stellen verwendet werden.

3. Verfahren nach Anspruch 1 oder 2, wobei die Position des Kopfes der Person gemessen wird und sich der Strabismuswinkel auf diese Position bezieht.

4. Gerät zum automatischen Bestimmen eines Strabismuswinkels der Augen einer Person, umfassend:
- mindestens eine erste Lichtquelle (L₁), um Licht auf die Augen einer Person zu richten,
- eine Bildgebungsvorrichtung (C₁, C₂), um einen Reflextest an beiden Augen auszuführen, um den Strabismuswinkel zu schätzen,
und ferner umfassend:
- zwei weitere Lichtquellen (L₂, L₃) an verschiedenen Positionen, wobei die Bildgebungsvorrichtung angeordnet ist, um die Hornhautreflexionen der Lichtquellen in beiden Augen zu messen,
- Rechenmittel, die an die Bildgebungsvorrichtung angeschlossen sind, um zu bewirken, dass:
- die Hornhautreflexionen verwendet werden, um die Koordinaten (CC_{L}, CC_{R}) des Hornhautmittelpunktes für die beiden Augen einzeln abzuleiten;
- für die beiden Augen die Koordinaten (PC_{L}, PC_{R}) des Pupillenmittelpunktes einzeln geschätzt werden;
- die Koordinaten des Hornhautmittelpunktes und die Koordinaten des Pupillenmittelpunktes der beiden Augen verwendet werden, um den Strabismuswinkel zu schätzen,
wobei:
- die Bildgebungsvorrichtung angeordnet ist, um den Lichtstrahl zu messen, der von den Augen reflektiert wird, um festzustellen, welches Auge der beiden Augen das fixierende ist, und
- das Rechenmittel angeordnet ist, um:
- sowohl für das fixierende Auge als auch für das nicht fixierende Auge die geschätzten Koordinaten des Hornhautmittelpunktes und die geschätzten Koordinaten des Pupillenmittelpunktes einzeln zu bestimmen, und um sowohl für das fixierende Auge als auch für das nicht fixierende Auge die optische Achse (OA_{L}, OA_{R}) durch den Hornhautmittelpunkt und den Pupillenmittelpunkt des betreffenden Auges festzustellen;
- für das fixierende Auge den Winkel Kappa zwischen der optischen Achse des fixierenden Auges und einer ersten Sichtachse (VA_{L}) durch den Hornhautmittelpunkt dieses fixierenden Auges und seine Sehgrube zu bestimmen;
- für das nicht fixierende Auge eine zweite Sichtachse (VA_{R}) aus der optischen Achse dieses nicht fixierenden Auges und dem Winkel Kappa, der für das fixierende Auge festgestellt wurde, abgeleitet wird, zu bestimmen;
- den Strabismuswinkel aus den so abgeleiteten ersten und zweiten Sichtachsen der beiden Augen zu bestimmen.

5. Gerät nach Anspruch 4, wobei die Bildgebungsvorrichtung zur Stereobildgebung angeordnet ist.

6. Gerät nach Anspruch 5, wobei die Bildgebungsvorrichtung mindestens zwei Kameras an verschiedenen Stellen umfasst.

## Revendications

1. Procédé pour déterminer automatiquement un angle de strabisme des yeux d'un individu par la réalisation d'un test de réflexion sur lesdits yeux, comprenant les étapes consistant à :
- diriger au moins un premier faisceau de lumière sur lesdits yeux de l'individu ;
- faire en sorte que l'individu fixe son regard ;
- utiliser un dispositif d'imagerie pour réaliser un test de réflexion sur les deux yeux afin d'estimer l'angle de strabisme,
dans lequel :
- le test de réflexe est réalisé en appliquant au moins deux sources lumineuses dans des positions différentes connues et en mesurant les réflexions cornéennes desdites sources lumineuses dans les deux yeux par la surveillance desdites réflexions cornéennes grâce à un ou des dispositifs d'imagerie ;
- lesdites réflexions cornéennes sont utilisées pour dériver les coordonnées du centre de la cornée (CC_{L} , CC_{R}) pour chaque oeil;
- pour chaque oeil séparément, les coordonnées du centre de la pupille (AC_{L}, PC_{R}) sont estimées ; et
- les coordonnées du centre de la cornée et les coordonnées du centre de la pupille des deux yeux servent à estimer l'angle de strabisme,
- tout d'abord, le faisceau lumineux réfléchi depuis les yeux est mesuré dans le cadre d'un premier test pour déterminer lequel des deux yeux est l'oeil fixateur, puis :
- pour chaque oeil, l'oeil fixateur et l'oeil non fixateur séparément, les coordonnées estimées du centre de la cornée et les coordonnées estimées du centre de la pupille servent à déterminer l'axe optique de chaque oeil (OA_{L}, OA_{R}) passant par le centre de la cornée et le centre de la pupille de l'oeil concerné ;
- pour l'oeil qui fixe, l'angle kappa est déterminé entre ledit axe optique de l'oeil fixateur et un premier axe visuel (VA_{L}) traversant le centre cornéen de l'oeil et sa fovea ;
- pour l'oeil qui ne fixe pas, un second axe visuel (VA_{R}) est dérivé dudit axe optique de l'oeil non fixateur et l'angle kappa qui est établi pour l'oeil fixateur ;
- l'angle du strabisme est dérivé des premier et second axes visuels des deux yeux ainsi dérivés.

2. Procédé selon la revendication 1, dans lequel la surveillance des réflexions cornéennes par le ou les dispositifs d'imagerie est réalisée à l'aide d'au moins de deux caméras situées à des endroits différents.

3. Procédé selon la revendication 1 ou 2, dans lequel la position de la tête de l'individu est mesurée et l'angle de strabisme est mise en rotation avec ladite position.

4. Équipement pour déterminer automatiquement un angle de strabisme des yeux d'un individu, comprenant :
- au moins une première source de lumière (L₁) pour diriger la lumière sur les yeux de l'individu,
- un dispositif d'imagerie (C₁, C₂) pour réaliser un test réflexe sur les deux yeux afin d'estimer l'angle de strabisme, et comprenant en outre :
- deux autres sources de lumière (L₂, L₃) dans des positions différentes, le dispositif d'imagerie étant agencé de façon à mesurer les réflexions cornéennes desdites sources lumineuses dans les deux yeux,
- des moyens de calcul connectés au dispositif d'imagerie de manière que :
- lesdites réflexions cornéennes sont utilisées pour dériver les coordonnées du centre de la cornée (CC_{L}, CC_{R}) pour chaque oeil séparément ;
- pour chaque oeil séparément, les coordonnées du centre de la pupille (PC_{L}, PC_{R}) sont estimées ; et
- les coordonnées du centre de la cornée et les coordonnées du centre de la pupille des deux yeux sont utilisées pour estimer l'angle de strabisme,
dans lequel :
- le dispositif d'imagerie est agencé de façon à mesurer le faisceau lumineux réfléchi depuis les yeux afin d'établir lequel des deux yeux est l'oeil fixateur, et
- les moyens de calcul sont agencés de façon à déterminer :
. pour chacun des deux yeux, celui qui fixe et celui qui ne fixe pas séparément, les coordonnées estimées du centre de la cornée et les coordonnées estimées du centre de la pupille, et à établir, pour les deux yeux, celui qui fixe et celui qui ne fixe pas, l'axe optique (OA_{L}, OA_{R}) traversant le centre de la cornée et le centre de la pupille de l'oeil concerné ;
. pour l'oeil qui fixe, l'angle kappa entre l'axe optique de l'oeil fixateur et un premier axe visuel (VAL) passant par le centre cornéen de l'oeil fixateur et sa fovea ;
. pour l'oeil qui ne fixe pas, un second axe visuel (VA_{R}) dépendant dudit axe optique de l'oeil non fixateur et l'angle kappa qui est établi pour l'oeil fixateur ;
. l'angle de strabisme des premier et second axes visuels des deux yeux ainsi dérivés.

5. Équipement selon la revendication 4 dans lequel le dispositif d'imagerie est agencé pour l'imagerie stéréoscopique.

6. Équipement selon la revendication 5 dans lequel le dispositif d'imagerie comprend au moins deux caméras placées à des endroits différents.
